# EUROPÄISCHE PATENTSCHRIFT

(11) **EP 0 777 714 B1**
(45) Veröffentlichungstag und Bekanntmachung des Hinweises auf die Patenterteilung: **20.01.1999**
(21) Anmeldenummer: 95929886.0
(22) Anmeldetag: 16.08.1995
(51) Int. Cl.: C11D 1/83, C11D 3/32, A61K 7/06

(54) **HAARSHAMPOOS**
HAIR SHAMPOOS
SHAMPOOINGS POUR LES CHEVEUX

(30) Priorität: 25.08.1994 DE 4430085
(43) Veröffentlichungstag der Anmeldung: 11.06.1997
(73) Patentinhaber: Henkel Kommanditgesellschaft auf Aktien, 40191 Düsseldorf (DE)
(72) Erfinder: FABRY, Bernd, D-41352 Korschenbroich (DE); KAHRE, Jörg, D-40789 Monheim (DE)
(86) Internationale Anmeldenummer: EP9503259
(87) Internationale Veröffentlichungsnummer: WO9606150

(56) Entgegenhaltungen:
- EP-A- 0 522 206
- WO-A-94/09099
- DE-A- 4 229 442

## Beschreibung

### Gebiet der Erfindung

Die Erfindung betrifft Haarshampoos mit synergistischer Verstärkung des Reinigungs- und Schaumvermögens, enthaltend Fettsäure-N-alkylpolyhydroxyalkylamide und Alkyl- und/oder Alkenyl(ether)phosphate.

### Stand der Technik

Fettsäure-N-alkylpolyhydroxyalkylamide und insbesondere Fettsäure-N-methylglucamide stellen nichtionische Tenside dar, die wegen ihres guten anwendungstechnischen Profils beispielsweise zur Herstellung von Handgeschirrspülmitteln eingesetzt werden. Die Verwendung dieser Stoffe ist Gegenstand einer Vielzahl von Veröffentlichungen. Aus der Europäischen Patentanmeldung **EP-A1 0285768** (Hüls) ist beispielsweise ihr Einsatz als Verdickungsmittel bekannt. In der Französischen Offenlegungsschrift **FR-A 1580491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Gegenstand der Internationalen Patentanmeldungen **WO 92/06153**; **WO 92/06156; WO 92/06157; WO 92/06158; WO 92/06159** und **WO 92/06160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/06152**; **WO 92/06154**; **WO 92/06155; WO 92/06161; WO 92/06162; WO 92/06164; WO 92/06170; WO 92/06171** und **WO 92/06172** (Procter & Gamble) beschrieben. In der Praxis hat sich jedoch gezeigt, daß die Fettsäure-N-alkylpolyhydroxyalkylamide auch in Kombination mit anderen vorzugsweise anionischen Tensiden ein Reinigungs- und Schaumvermögen aufweisen, das nicht immer zufriedenstellend sind. Mischungen von Fettsäureglucamiden mit stickstofffreien Tensiden, die dennoch über gute avivierende Eigenschaften verfügen, sind ebenfalls nicht bekannt.

Die Aufgabe der Erfindung hat somit darin bestanden, auf Basis von Fettsäure-N-alkylpolyhydroxyalkylamiden zur Verfügung zu stellen, die über ein verbessertes Reinigungs-, Schaum- und Avivagevermögen verfügen, ohne daß dieser Vorteil durch Leistungseinbußen in anderen anwendungs technisch relevanten Eigenschaften bezahlt werden muß.

### Beschreibung der Erfindung

Gegenstand der Erfindung sind Haarshampoos mit verbesserter Reinigungsleistung, enthaltend 10 bis 30 Gew.-% - bezogen auf die Mittel -
(a) Fettsäure-N-alkylpolyhydroxyalkylamide und
(b) Alkyl- und/oder Alkenyl(ether)phosphate
im Gewichtsverhältnis 80 : 20 bis 20 : 80 und vorzugsweise 50 : 50 bis 25 : 75.

Obschon Mischungen auf Basis von Fettsäure-N-alkylpolyhydroxyalkylamiden, insbesondere Fettsäure-N-methylglucamiden und anionischen Tensiden bereits bekannt gewesen sind, wurde überraschenderweise hinsichtlich des Reinigungs- und Schaumvermögens innerhalb bestimmter Mischungsverhältnisse eine starke synergistische Wechselwirkung mit Alkyl- und/oder Alkenyl(ether)phosphaten festgestellt. Die Erfindung schließt die Erkenntnis ein, daß auch hinsichtlich Netzvermögen und Waschleistung eine Verbesserung erzielt wird und die ökotoxikologischen Eigenschaften wenigstens auf dem Niveau der Einzelstoffe erhalten bleiben. Als besonders überraschend hat sich erwiesen, daß der Einsatz insbesondere von ungesättigten Etherphosphaten in Haarreinigungs- und -pflegemitteln im Vergleich zu Produkten, die typische kationische Tenside als Avivagekomponenten enthalten, zu einer Verbesserung der Naß- und Trockenkämmbarkeit führt.

### Fettsäure-N-alkylpolyhydroxyalkylamide

Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf.Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide steilen daher **Fettsäure-N-alkylglucamide** dar, wie sie durch die Formel **(II)** wiedergegeben werden:

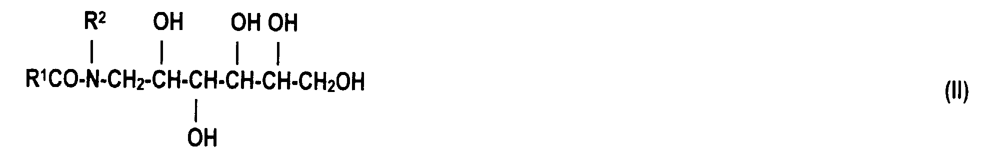

Vorzugsweise werden als Fettsäure-N-alkylpolyhydroxyalkylamide Glucamide der Formel **(II)** eingesetzt, in der R² für Wasserstoff oder eine Amingruppe steht und R¹CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(II)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Alkyl- und(oder) Alkenyl(ether)phosphate

Alkyl- und/oder Alkenylphosphate bzw. -etherphosphate stellen bekannte anionische Tenside dar, die üblicherweise durch Umsetzung von gegebenenfalls ethoxylierten gesättigten und/oder ungesättigten Fettalkoholen mit Phosphorpentoxid hergestellt werden. In Abhängigkeit des Einsatzverhältnisses der Komponenten werden dabei technische Gemische erhalten, die überwiegend Mono- und Dialkylester, neben Triestern, Phosphorsäure und nicht umgesetzter Alkoholkomponente enthalten. Übersichten zu diesem Thema sind beispielsweise von R.S.Cooper sowie G.lmokawa in **J.Am.Oil.Chem.Soc. 41, 337 (1964)** bzw. **55, 839 (1978)**, H.Distler in **Tens.Detergents 12, 263 (1975)** und O'Lennick in **Soap, Cosm.,Chem.Spec. 7, 26 (1986)** erschienen. Alkyl- und/oder Alkenyl(ether)phosphate, die im Sinne der Erfindung in Betracht kommen, folgen der Formel **(III)**, 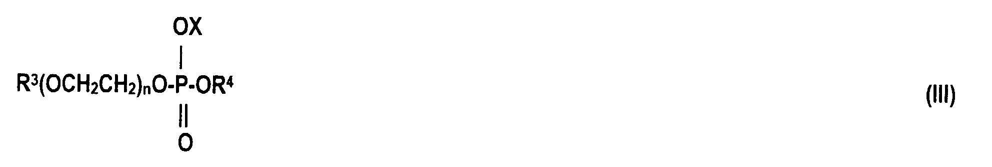 in der R³ für einen linearen oder verzweigten Alkylrest mit 6 bis 22 Kohlenstoffatomen, R⁴ für eine Gruppe R¹(OCH₂CH₂)ₙ oder X, n für 0 oder Zahlen von 1 bis 10 und X für ein Alkali- und/oder Erdalkalimetall, Ammonium, Alkylammonium, Alkanolammonium oder Glucammonium steht. Typische Beispiele sind Oleylphosphat sowie Mono- und/oder Dialkylester auf Basis von Addukten von 1 bis 10, vorzugsweise 2 bis 8 Mol Ethylenoxid an Capronalkohol, Caprylalkohol, 2-Ethylhexylalkohol, Caprinalkohol, Laurylalkohol, lsotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Besonders bevorzugt ist der Einsatz von Alkyl(ether)phosphaten mit einem Veresterungsgrad im Bereich von 1 bis 2,5 und vorzugsweise 1,3 bis 2,1 auf Basis von Anlagerungsprodukten von 1 bis 5 Mol Ethylenoxid an 2-Ethylhexanol, sowie C_{12/14}-bzw. C_{12/18}-Kokosfettalkohol und insbesondere technische Oleylalkohole im lodzahlbereich 45 bis 115, vorzugsweise 55 bis 95.

### Haarshampoos

Die Herstellung der Haarshampoos erfolgt in der Regel durch Verrühren wäßriger Lösungen, Pasten oder Konzentrate der Einsatzstoffe, falls erforderlich bei leicht erhöhter Temperatur. Es ist jedoch ebenfalls möglich, sprühgetrocknete Pulver der beiden Komponenten zu vermischen und anschließend anzupasten oder Lösungen beider Inhaltsstoffe gemeinsam einer Sprühtrocknung und/oder Granulation zu unterwerfen. In allen diesen Fällen handelt es sich um rein mechanische Verfahren, eine chemische Reaktion findet nicht statt.

### Gewerbliche Anwendbarkeit

Die erfindungsgemäßen Detergensgemische weisen eine synergistische Verstärkung des Reinigungs- und Schaumvermögens auf.

### Tenside

Die Haarshampoos können als Hilfs- und Zusatzstoffe in erster Linie weitere anionische, nichtionische, kationische, amphotere und/oder zwitterionische Tenside enthalten. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Alkyloligoglucosidsulfate, Proteinfettsäurekondensate (insbesondere pflanzliche Produkte auf Sojabasis), Sulfosuccinate und Sulfosuccinamate. Sofern die anionischen Tenside Polyglycoletherketten enthalten, können sie eine konventionelle, vorzugsweise jedoch eine eingeengte Homologenverteilung aufweisen. Typische Beispiele **für nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Alk(en)yloligoglykoside, Fettsäure-N-alkylglucamide, Proteinhydrolysate (insbesondere pflanzliche Produkte auf Sojabasis) Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, lmidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Weitere Hilfs- und Zusatzstoffe

Die Haarshampoos können als Hilfs- und Zusatzstoffe **Emulgatoren** wie etwa alkoxylierte Fettalkohole oder Sorbitanester enthalten. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmonound -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate und ähnliche Verbindungen. Als **Konservierungsmittel** eignen sich beispielsweise Phenoxyethanol, Formaldehydlösung, Parabene, Pentadiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farb** **stoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

Die Untersuchung des Schaumvermögens erfolgte gemäß Ross-Miles-Test DIN 53901, Teil 2) in einer 1 Gew.-%igen wäßrigen Lösung (Wasserhärte 16°d, Temperatur 20°C). Die Ergebnisse zu Basisschaum und Schaumzerfall sind in Tabelle 1 zusammengefaßt (Prozentangaben als Gew.-%). Die Beispiele 1 bis 9 sind erfindungsgemäß, die Beispiele V1 bis V8 dienen zum Vergleich.

**Tabelle 1**

| **Basisschaum und Schaumzerfall** | | | | | | | |
|---|---|---|---|---|---|---|---|
| **Bsp.** | **A** | **B1** | **B2** | **C** | **D** | **Schaumhöhe [ml]** | |
| | | | | | | **Basis** | **nach 5 min** |
| 1 | 80 | 20 | - | - | - | 510 | 420 |
| 2 | 70 | 30 | - | - | - | 520 | 410 |
| 3 | 60 | 40 | - | - | - | 530 | 420 |
| 4 | 50 | 50 | - | - | - | 540 | 440 |
| 5 | 40 | 60 | - | - | - | 570 | 460 |
| 6 | 40 | - | 60 | - | - | 550 | 430 |
| 7 | 30 | 70 | - | - | - | 560 | 460 |
| 8 | 25 | 75 | - | - | - | 560 | 460 |
| 9 | 20 | 80 | - | - | - | 500 | 430 |
| V1 | 100 | - | - | - | - | 450 | 325 |
| V2 | - | 100 | - | - | - | 400 | 325 |
| V3 | - | - | 100 | - | - | 325 | 300 |
| V4 | - | - | - | 100 | - | 600 | 280 |
| V5 | - | - | - | - | 100 | 550 | 350 |
| V6 | 40 | - | - | 60 | - | 500 | 300 |
| V7 | 40 | - | - | - | 60 | 475 | 300 |
| V8 | 0 | 60 | - | 40 | - | 500 | 400 |
| **Legende:** (A) C_{12/14}-Kokosfettsäure-N-methylglucamid; (B1) C_{12/14}-Kokosalkyl+3EO-phosphat; Verhältnis Mono/diphosphat = 2:1; (B2) Oleyl+3EO-phosphat, Verhältnis Mono/diphosphat 2:1, Reinheit bezogen auf Oleylalkohol ca. 85 Gew.-%; (C) Dodecylbenzolsulfonat-Na-Salz; (D) C_{12/14}-Kokosfettalkohol+7EO-ethercarboxylat-Na-Salz | | | | | | | |

## Patentansprüche

1. Haarshampoos, **dadurch gekennzeichnet,** daß sie 10 bis 30 Gew.-% - bezogen auf die Mittel -
(a) Fettsäure-N-alkylpolyhydroxyalkylamide und
(b) Alkyl- und/oder Alkenyl(ether)phosphate
im Gewichtsverhältnis 80 : 20 bis 20 : 80 enthalten.

2. Haarshampoos nach Anspruch 1, **dadurch gekennzeichnet,** daß sie als Komponente (a) Fettsäure-N-alkylpolyhydroxyalkylamide der Formel **(I)** enthalten, 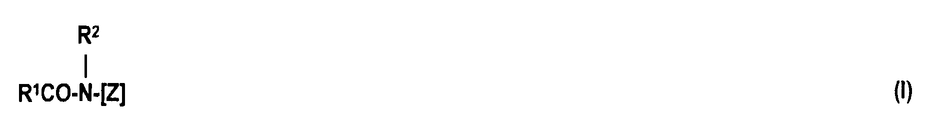 in der R¹CO für einen aliphatischen Acylrest mit 6 bis 22 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

3. Haarshampoos nach Anspruch 2, **dadurch gekennzeichnet,** daß sie als Komponente (a) Fettsäure-N-alkylglucamide enthalten.

4. Haarshampoos nach den Ansprüchen 1 bis 3, **dadurch gekennzeichnet,** daß sie als Komponente
(b) Alkyl- und/oder Alkenyl(ether)phosphate der Formel **(III)** enthalten, 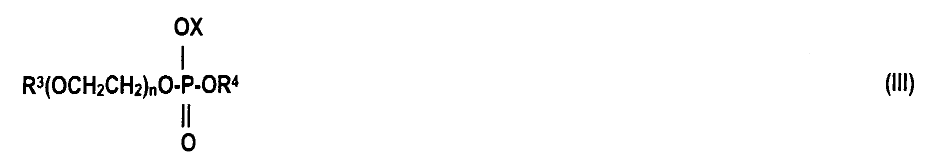 in der R³ für einen linearen oder verzweigten Alkylrest mit 6 bis 22 Kohlenstoffatomen, R⁴ für eine Gruppe R¹(OCH₂CH₂)ₙ oder X, n für 0 oder Zahlen von 1 bis 10 und X für ein Alkali- und/oder Erdalkalimetall, Ammonium, Alkylammonium, Alkanolammonium oder Glucammonium steht.

5. Haarshampoos nach den Ansprüchen 1 bis 4, **dadurch gekennzeichnet,** daß sie die Komponenten (a) und (b) im Gewichtsverhältnis 50 : 50 bis 25 : 75 enthalten.

## Claims

1. Hair shampoos, characterized in that they contain 10 to 30% by weight, based on the shampoo, of
(a) fatty acid-N-alkyl polyhydroxyalkylamides and
(b) alkyl and/or alkenyl (ether) phosphates
in a ratio by weight of 80:20 to 20:80.

2. Hair shampoos as claimed in claim 1, characterized in that they contain as component (a) fatty acid-N-alkyl polyhydroxyalkylamides corresponding to formula (I): 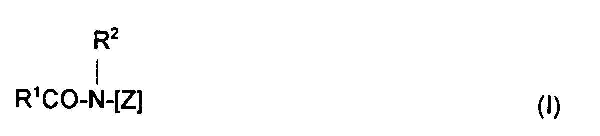 in which R¹CO is an aliphatic acyl group containing 6 to 22 carbon atoms, R² is an alkyl or hydroxyalkyl group containing 1 to 4 carbon atoms and [Z] is a linear or branched polyhydroxyalkyl group containing 3 to 12 carbon atoms and 3 to 10 hydroxyl groups.

3. Hair shampoos as claimed in claim 2, characterized in that they contain fatty acid-N-alkyl glucamides as component (a).

4. Hair shampoos as claimed in claims 1 to 3, characterized in that they contain as component (b) alkyl and/or alkenyl (ether) phosphates corresponding to formula (III): 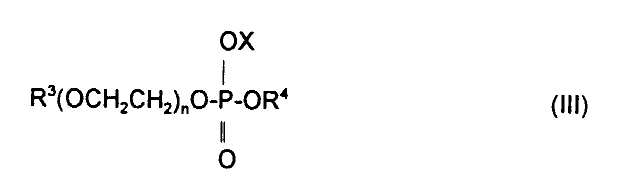 in which R³ is a linear or branched alkyl group containing 6 to 22 carbon atoms, R'is a group R¹(OCH₂CH₂)ₙ or X, n is 0 or a number of 1 to 10 and X is an alkali metal and/or alkaline earth metal, ammonium, alkyl ammonium, alkanolammonium or glucammonium.

5. Hair shampoos as claimed in claims 1 to 4, characterized in that they contain components (a) and (b) in a ratio by weight of 50:50 to 25:75.

## Revendications

1. Shampooings pour cheveux
caractérisés en ce qu'
ils contiennent de 10 à 30 % en poids, par rapport aux agents
a) amides d'acide gras-N-alkylpolyhydroxyalkilés,
b) (éther) phosphates d'alkyle et/ou d'alkényle
dans le rapport pondéral 80:20 à 20:80.

2. Shampooings pour cheveux selon la revendication 1,
caractérisés en ce qu'
ils contiennent comme composant (a) des amides d'acide gras-N-alkylpolyhydroxyalkilés de la formule (I) 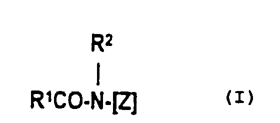 dans laquelle R¹CO représente un radical acyle aliphatique avec 6 à 22 atomes de carbone, R² un radical alkyle ou hydroxyalkyle avec 1 à 4 atomes de carbone et (Z) un radical polyhydroxyalkyle avec 3 à 12 atomes de carbone et 3 à 10 groupes hydropxyle.

3. Shampooings pour cheveux selon la revendication 2,
caractérisés en ce qu'
ils contiennent comme composant (A) des glucamides d'acide gras-N-alkylés.

4. Shampooings pour cheveux selon les revendications 1 à 3,
caractérisés en ce qu'
ils contiennent comme composant (b) des (éther) phosphates d'alkyle et/ou d'alcényle de la formule (III) 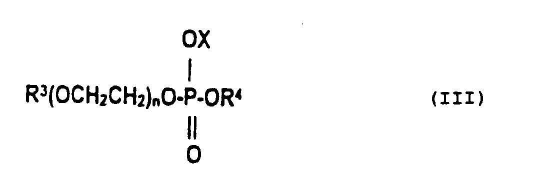 dans laquelle
R³ remplace un radical alkyle linéaire ou ramifié avec 6 à 22 atomes de carbone, R⁴ un groupe R¹(OCH₂CH₂)ₙ ou X, n remplace 0 ou des nombres de 1 à 10 et X un métal alcalin et/ou alcalino-terreux, de l'ammonium, de l'alkylammonium, de l'alcanolammonium ou du glucomonium.

5. Shampooings pour cheveux selon les revendications 1 à 4,
caractérisés en ce qu'
ils contiennent les composants (a) et (b) dans le rapport de poids 50:50 à 25:75.
